# EUROPEAN PATENT APPLICATION

(11) **EP 4 434 451 A1**
(43) Date of publication of application: **25.09.2024**
(21) Application number: 24163615.8
(22) Date of filing: 14.03.2024
(51) Int. Cl.: A61B 5/024, A61B 5/00, A61B 5/08

(54) **OPTICAL RESPIRATION RATE PREDICTION SYSTEM**

(30) Priority: 20.03.2023 US 202318123694
(71) Applicant: Analog Devices, Inc., Wilmington, MA 01887 (US)
(72) Inventor: Liu, Jing, Wilmington, 01887 (US); Foroozan, Foroohar, Wilmington, 01887 (US)
(74) Representative: Wallin, Nicholas James

(57) **Abstract**

A respiration rate prediction system and method can include: emitting light into tissue with a light emitter; detecting the light from the tissue with a photodetector as a hemodynamic signal; determining a respiration rate estimate and a signal condition feature from the hemodynamic signal; determining a signal quality indicator from the signal condition feature; deciding whether to determine a respiration rate prediction with a machine learning algorithm based on the signal quality indicator; determining the respiration rate prediction and a confidence score for the respiration rate prediction, the confidence score based on the respiration rate estimate and the signal condition feature being inputs into the machine learning algorithm; and outputting the respiration rate prediction from the machine learning algorithm based on the confidence score.

## Description

### TECHNICAL FIELD

This disclosure relates to systems generating respiration rate information, more particularly to the use of photoplethysmography (PPG) for predicting respiration rate from the wrist or other parts of the body with suboptimal anatomy or vascularity.

### BACKGROUND

As the most sensitive predictor of patient deterioration, respiration rate (RR) and its assessment are critical in intensive care unit admission, diagnostic testing of apnea, and detecting obstructive pulmonary disease exacerbations. Amid the COVID-19 pandemic, RR measurement has become even more relevant in respiratory management.

In traditional clinical practice, RR measurement is performed by manually counting the chest wall movements along with respirations, which is labor-intensive and intermittent. Precise and continuous RR monitoring can be performed with capnography via nasal or oral cannula, where RR is detected from the respiration cycle lengths in the measured end-tidal CO2 signal. However, capnographic measurement is not practiced in routine examination and self-health monitoring due to its complicated and cumbersome setup, its expensive equipment, its intensive labor requirements, and its constrictive and uncomfortable feel for a patient.

Therefore, wearable devices enabling convenient and effective RR monitoring is highly demanded. PPG relies on simple light-emitting diodes (LEDs) and photodiodes, and PPG is a low-cost and widespread optical technique for detecting variations of blood volume in blood containing tissue.

PPG sensors have been widely adopted in various wearable devices for heart rate monitoring. In fact, PPG signals are also modulated by respiration-induced variations in blood volume profiles, which makes it possible to use PPG signals for RR monitoring.

In recent years, wearable devices have been gaining popularity for daily and remote health monitoring. The PPG sensor composed of a simple LED and a photodetector is the most popular biosensor in smartwatches. The PPG signal can characterize blood volume variations in the rhythm of heartbeats; thus, it has been primarily used for heart rate monitoring, and there are also extensive efforts to fully exploit respiratory modulations contained within PPG signals.

Of all prior developments, only the prior fingertip PPG-based RR method has achieved clinical-grade accuracy. No other RR measurement for use on other parts of the body has been shown to provide the requisite accuracy.

Prior fingertip PPG-based RR developments achieve accurate RR readings with a finger-worn sensor, but the measurement accuracy is not stable enough to be widely acknowledged for applications at other locations on the body. The lower vascular density and anatomic differences at the wrist, for example, pose outstanding challenges for prior fingertip PPG-based RR developments as prior fingertip sensors do not return consistently accurate RR information when used on other parts of the body with suboptimal anatomies and vascularity.

Illustratively, prior fingertip PPG-based RR determinations are susceptible to motion artifacts from bone, tendon, and tissue which are frequently encountered in wrist measurements but are not encountered in fingertip measurements. Sparse and varicose distribution of major arteries and veins together with lower blood perfusion in the wrist, compared to fingertip, prevents prior fingertip PPG-based RR developments from accurately determining RR from the wrist.

Further complicating the prior fingertip PPG-based RR developments, a single-wavelength PPG is normally used. However, the PPG waveform is highly dependent on the underlying vasculature and several concurrent blood volume regulation mechanisms. The resultant uncertainty in direct estimations of RR from the single-wavelength PPG leads to unsatisfactory accuracy and difficulty in extracting RR information therefrom.

Though there are extensive efforts in estimating RR from PPG signals, the accuracy and working range of such PPG-based RR method has not been well-acknowledged for routine applications, and many confounding factors still remain. PPG, as a reflection of blood volume, is directly regulated by cardiovascular system and only indirectly affected by respiration through complicated cardiorespiratory coupling mechanisms.

Modulations in the PPG signal can also be caused by other non-respiratory factors such as neural-modulated vasomotion and muscle contractions. This limits the ability to provide a robust accurate determination of RR from suboptimal tissue.

Solutions have been long sought but prior developments have not taught or suggested any complete solutions, and solutions to these problems have long eluded those skilled in the art. Thus, there remains a considerable need for devices and methods that can provide a robustly accurate RR measurement from a device that can be used on the wrist or other parts of the body with suboptimal anatomy and vascularity.

### SUMMARY

An optical RR prediction system and methods, providing a robust and accurate RR measurement from the wrist or other parts of the body with suboptimal anatomy and vascularity, are disclosed. The RR prediction system and methods can include: emitting light into tissue with a light emitter; detecting the light from the tissue with a photodetector as a hemodynamic signal; determining a respiration rate estimate and a signal condition feature from the hemodynamic signal; determining a signal quality indicator from the signal condition feature; deciding whether to determine a respiration rate prediction with a machine learning algorithm based on the signal quality indicator; determining the respiration rate prediction and a confidence score for the respiration rate prediction, the confidence score based on the respiration rate estimate and the signal condition feature being inputs into the machine learning algorithm; and outputting the respiration rate prediction from the machine learning algorithm based on the confidence score.

Other contemplated embodiments can include objects, features, aspects, and advantages in addition to or in place of those mentioned above. These objects, features, aspects, and advantages of the embodiments will become more apparent from the following detailed description, along with the accompanying drawings.

### BRIEF DESCRIPTION OF THE DRAWINGS

The RR prediction system is illustrated in the figures of the accompanying drawings which are meant to be exemplary and not limiting, in which like reference numerals are intended to refer to like components, and in which:
FIG. 1 is an anatomical side view of the RR prediction system implemented in a watch on a wrist of a patient.
FIG. 2 is a bottom view of the RR prediction system of FIG. 1.
FIG. 3 is a cross-sectional view of the RR prediction system along the line 3-3 of FIG. 1.
FIG. 4 is a block diagram of the RR prediction system in a respiratory feature extraction phase of operation together with a chain of events during breathing activities.
FIG. 5 is a block diagram of a control flow for the RR prediction system.
FIG. 6 is a block diagram of a control flow for determining a heart rate variability (HRV) RR estimate.
FIG. 7 is a block diagram of a control flow for determining a wandering baseline RR estimate.

### DETAILED DESCRIPTION

In the following description, reference is made to the accompanying drawings that form a part hereof, and in which are shown by way of illustration, embodiments in which the RR prediction system may be practiced. It is to be understood that other embodiments may be utilized and structural changes may be made without departing from the scope of the RR prediction system.

When features, aspects, or embodiments of the RR prediction system are described in terms of steps of a process, an operation, a control flow, or a flow chart, it is to be understood that the steps can be combined, performed in a different order, deleted, or include additional steps without departing from the RR prediction system as described herein.

The RR prediction system is described in sufficient detail to enable those skilled in the art to make and use the RR prediction system and provide numerous specific details to give a thorough understanding of the RR prediction system; however, it will be apparent that the RR prediction system may be practiced without these specific details.

In order to avoid obscuring the RR prediction system, some well-known system configurations, algorithms, equations, and descriptions are not disclosed in detail. Likewise, the drawings showing embodiments of the system are semi-diagrammatic and not to scale and, particularly, some of the dimensions are for the clarity of presentation and are shown greatly exaggerated in the drawing FIGs.

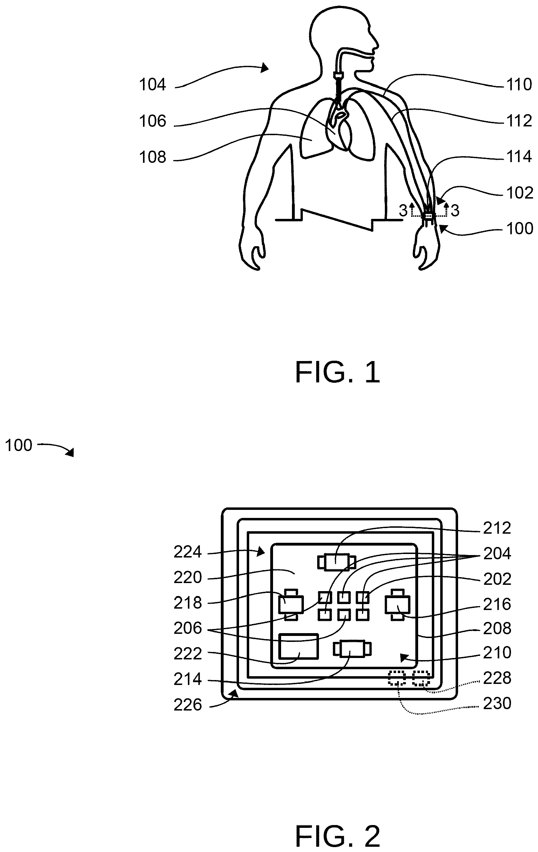

Referring now to FIG. 1, therein is shown an anatomical side view of the RR prediction system 100 implemented in a watch on a wrist 102 of a user 104. The RR prediction system 100 can be equipped with multi-wavelength PPG sensors, as set forth with regard to FIG. 2 below. The multi-wavelength PPG sensors can be in direct contact with the user 104 or can be spaced apart.

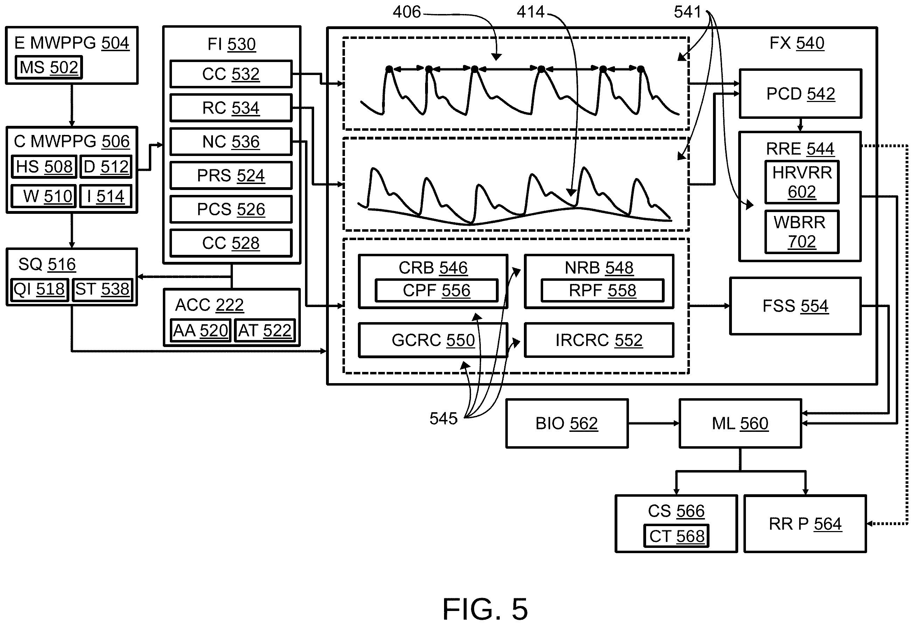

The multi-wavelength PPG sensors of the RR prediction system 100 are used to measure the user's 104 hemodynamic signals 508 of FIG. 5, after which features are extracted from the hemodynamic signals 508. A "feature" or "features" are data or relationships within data or between data, specifically data or relationships within or between the hemodynamic signals 508 collected by the RR prediction system 100.

The features can be multidimensional respiratory-induced features extracted from the multi-wavelength PPG hemodynamic signal 508. That is, the features can be modulations of the hemodynamic signals 508 due to cardiorespiratory coupling mechanisms and other non-respiratory factors such as neural-modulated vasomotion and muscle contractions. "Modulation" or "modulated" as used herein, means varying one or more properties of data, and when used in terms of respiration-modulated means varying one or more properties of data based on a respiration activity of the user 104.

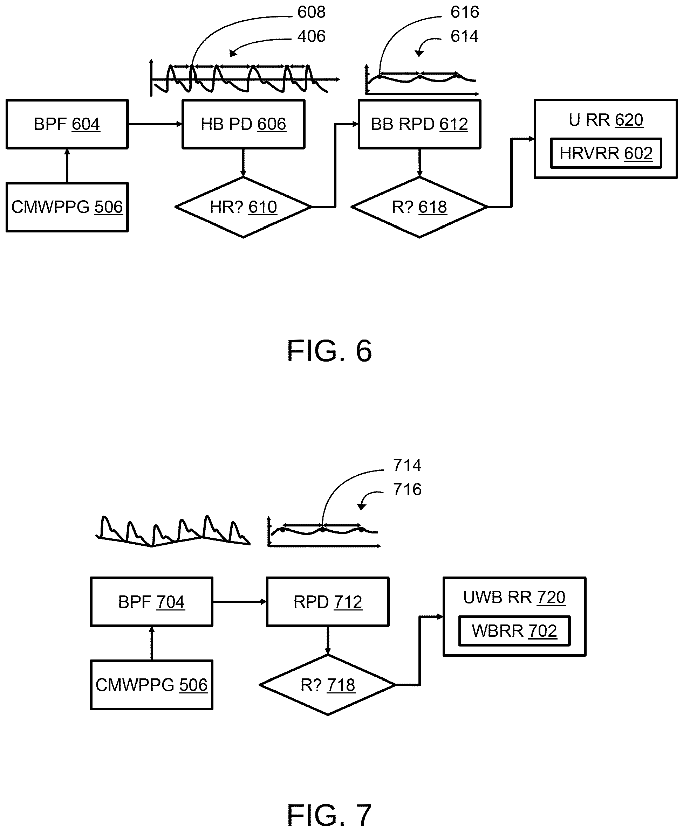

Continuing with the illustration of features extracted from the hemodynamic signals 508, features extracted during the feature extraction step 540 of FIG. 5 include the HRV RR estimate 602 of FIG. 6, the wandering baseline RR estimate 702 of FIG. 7, the cardio-respiratory balance 546 of FIG. 5, a neuro-respiratory balance 548 of FIG. 5, a green cardio-respiratory coupling 550 of FIG. 5, an IR cardio-respiratory coupling 552 of FIG. 5, and could also include others. The HRV RR estimate 602 and the wandering baseline RR estimate 702 can be understood as the respiratory features 541 of FIG. 5 while the cardio-respiratory balance 546, the neuro-respiratory balance 548, the green cardio-respiratory coupling 550, and the IR cardio-respiratory coupling 552 can be understood as the signal condition features 545 of FIG. 5. These features reflect the coupling between the cardiovascular, neurological, and respiratory modulations of the user 104.

Volumetric blood changes in the user 104 are directly modulated by the user's 104 heart 106 and lungs 108 and are indirectly modulated by respiration through complicated cardiorespiratory coupling mechanisms. As a result of the physiological and physical coupling between the cardiovascular and respiratory systems, respiratory activities induce changes of blood volume and pulse rate in arteries 110, veins 112, and capillaries 114.

These respiration-induced changes in blood volume can be transmitted to the peripheral vasculatures and measured with the multi-wavelength PPG sensor as amplitude fluctuations such as baseline wandering, and frequency domain variations such as heart rate variability.

The multi-wavelength PPG sensors can provide depth-resolved measurements from different blood vessels at different depths enabling more robust and comprehensive respiration picture to be captured even in areas of the user 104 that have suboptimal tissue vasculature, such as in the wrist 102. Similarly, the signal condition features 545 extracted from the multi-wavelength PPG signal, as described in the feature extraction step 540, are used to mitigate uncertainty in the respiratory features 541, which include the HRV RR estimate 602 and the wandering baseline RR estimate 702. The signal features 545 reflect the coupling status between the heart 196 and the lungs 108 of the user 104.

Furthermore, the respiratory features 541 and the signal condition features 545 can be provided as inputs to a Gaussian process regression for a final determination of the RR prediction 564 of FIG. 5 and confidence score 566 of FIG. 5. The Gaussian process regression is a non-parametric Bayesian approach that provides uncertainty estimates for its predictions.

The Gaussian process regression algorithm is suitable to handle the multi-wavelength PPG based RR predictions, considering the uncertainty in divergent RR estimates from different channels and the covariances with the physiological coupling. The RR prediction system 100 is therefore equipped to utilize physiological-informed signal condition features 545 and respiratory features 541 together with the Gaussian process regression, which when used together have shown to achieve accuracy in root mean squared error below 3 respirations per minute.

It has been discovered that this accuracy is equivalent to the accuracy from fingertip measurements and represents an improvement to RR detection technology enabling robust and accurate RR determinations from suboptimal areas of the user 104, such as the wrist.

Referring now to FIG. 2, therein is shown a bottom view of the RR prediction system 100 of FIG. 1. The multi-wavelength PPG sensor of the RR prediction system 100 is shown having light emitters of multiple wavelengths and photodetectors.

The light emitters are contemplated to be solid state light emitters, or light emitters manufactured using integrated circuit manufacturing techniques, such as light emitting diodes (LED) or laser diodes. For descriptive clarity only, the light emitters will be described herein with reference to LEDs.

The optical sensing layout of the RR prediction system 100 can therefore include light emitters for multiple wavelength bands in the wavelength bands of green light, red light, and infrared light. More particularly, the RR prediction system 100 can for example include one or multiple light emitters for each wavelength, as is illustrated by: a green LED 202, three red LEDs 204, and two infrared (IR) LEDs 206. The LEDs can be distributed in a 2X6 grid and exposed from an aperture 208 in the bottom surface 210 of the RR prediction system 100.

Peripheral to the LEDs and exposed from within the aperture 208, the RR prediction system 100 can include photodetectors which can be multi-wavelength and configured to detect the type of light emitted by the light emitters. The photo detectors are contemplated to be solid state photodetectors such as photodiodes, reverse biased LEDs, or even arrays of linked or coupled capacitors.

In one contemplated embodiment, the photodetectors are arranged to correspond with the four cardinal directions when looking at the RR prediction system 100 and facing north. That is, the RR prediction system 100 can include an east photodetector 212, a west photodetector 214, a north photodetector 216, and a south photodetector 218.

The LEDs and the photodetectors can be affixed to a substrate 220 together with an accelerometer 222. The accelerometer 222 can provide measurements of the RR prediction system's 100 acceleration.

The LEDs and the photodetectors can be protected and isolated with an optically transparent cover 224 such as a clear polymer encapsulation, or clear polymer or glass cover. The substrate 220 and the cover 224 can be affixed to a body 226 of the RR prediction system 100.

The RR prediction system 100 can further include non-transitory computer readable medium 228 in useful association with a processor 230. The computer readable medium 228 can have instructions and together, in useful association with the processor 230, can be configured to control all technical process and internal functioning of the RR prediction system 100 itself and its interfaces.

Illustratively for example, the computer readable medium 228 can have instructions configured to control the precise illumination of the LEDs to emit the multi-wavelength PPG signals 502 of FIG. 5. The computer readable medium 228 can have instructions further configured to detect and analyze the hemodynamic signals 508 of FIG. 5.

This detection and analysis cannot practically be performed in the human mind, because for instance, the human mind is not equipped to perform the signal analysis in real time or even on the operation window 510 of FIG. 5, and as a practical matter, cannot be performed entirely in a human's mind.

The methods of signal analysis with respect to the feature extraction step 540 of FIG. 5 and the filter step 530 of FIG. 5 require the manipulation of computer data structures (e.g., the modulated hemodynamic signals 508 from a multi-wavelength PPG array) and the output of a modified computer data structure of the RR prediction 564 of FIG. 5 and the confidence score 566 of FIG. 5.

As such, the disclosed solution is necessarily rooted in PPG technology by setting forth improvements to PPG methods and system configurations in order to overcome the problem of low accuracy measurements in areas of the body with suboptimal vascularity, which is a problem specifically arising in the realm of prior PPG solutions, and which could only obtain a sufficient accuracy from fingertip measurements with optimal vascularity. The disclosed improvement therefore solves a specific technical problem by technical means.

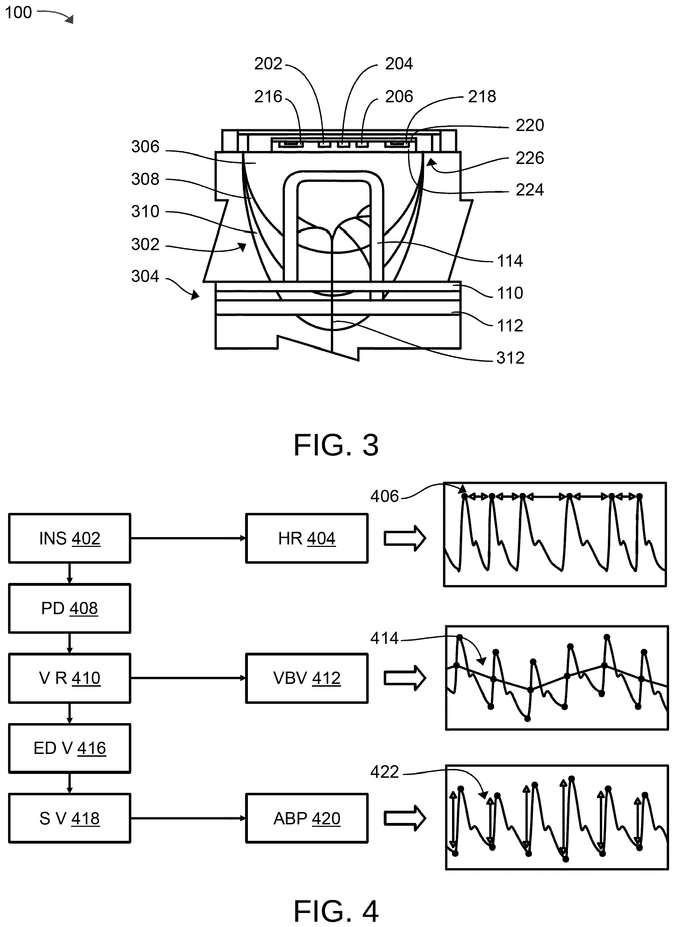

Referring now to FIG. 3, therein is shown a cross-sectional view of the RR prediction system 100 along the line 3-3 of FIG. 1. The RR prediction system 100 is shown with the multi-wavelength PPG sensor having the green LED 202, the red LED 204, and the IR LED 206 between the north photodetector 216 and the south photodetector 218.

The LEDs and the photodetectors are mounted to the substrate 220 and encapsulated with the cover 224. The multi-wavelength PPG sensor itself can be affixed to the body 226 of the RR prediction system 100.

The multi-wavelength PPG sensor is depicted in direct contact with the wrist 102 of the user 104 of FIG. 1 but could be offset from the skin. Complicated vascular structures are shown within the wrist 102 and complicate optical PPG measurements.

Illustratively, the wrist 102 will generally have a lower perfusion of blood compared to finger. Major arteries 110 and veins 112 can be sparse and varicose creating significant intra-individual and inter-individual variances in both an optical path 302 and a vascular path 304.

Since the light penetration depth into the skin depends on its wavelength, the multi-wavelength PPG sensor can provide depth-resolved pulsation measurements from different blood vessels at different depths, thus enabling more profound and comprehensive determination of respiration modulations in the various different portions of the vascular path 304. That is, the hemodynamic signals 508 of FIG. 5 can be detected at different depths within the user 104 and each depth can provide additional and different data features arising from a patient's respiration response in different depths of skin and blood vessels, which can be used for determining the RR prediction 564 of FIG. 5. It has been discovered that computing these data features, including the determination of the signal condition features 545 of FIG. 5, provide a technical solution which enables the accurate estimation of RR in areas of suboptimal vasculature.

The green LED 202 can provide a green optical path 306, the red LED 204 can provide a red optical path 308, and the IR LED 206 can provide the IR optical path 310. In relation to the other LEDs, the green LED 202 can be considered a short wavelength LED with a shallow optical path, the red LED 204 can be considered a medium wavelength LED with a medium optical path, and the IR LED 206 can be considered a long wavelength LED with a deep optical path.

The RR prediction system 100 can therefore measure blood pulsation information from different blood vessels at different depths within the wrist 102 of the user 104. For example, the green LED 202 can return measurements from the capillaries 114 and superficial micro-vessels which are distributed shallowly within the green optical path 306.

The red LED 204 can return measurements from the veins 112, the arteries 110, and the capillaries 114 distributed within the red optical path 308. The IR LED 206 can return measurements from the veins 112, the arteries 110, and the capillaries 114 distributed within the IR optical path 310.
multi-wavelength PPG signal measurements from the green optical path 306, the red optical path 308, and the IR optical path 310 can each be modulated by the physiological processes within the user 104 and can be detected by the photodetectors as a channel of the hemodynamic signals 508 with each channel corresponding to a different optical path. Some respiratory modulations within the hemodynamic signals 508 are manifested in PPG waveforms as amplitude fluctuations like baseline wandering and frequency domain variations like HRV

Other modulations within the hemodynamic signals 508 can occur from neural-modulated vasomotion and muscle contractions. These modulations are likely to happen in blood vessels innervated with sympathetic nerves 312, such as the capillaries 114, the veins 112, and within the wrist 102.

These respiratory modulations within the hemodynamic signals 508 can be extracted as respiratory features, such as the HRV RR estimate 602 of FIG. 6 and the wandering baseline RR estimate 702 of FIG. 7, which are approximations of respiration rate. Each PPG channel can detect its own respiratory features potentially providing a unique RR estimate for the green optical path 306, the red optical path 308, and the IR optical path 310.

Referring now to FIG. 4, therein is shown a block diagram of the RR prediction system 100 in a respiratory feature extraction phase of operation together with a chain of events during breathing activities. PPG as a reflection of blood volume is directly regulated by cardiovascular system and indirectly affected by respiration through complicated cardiorespiratory coupling mechanisms.

Beginning with an initial breath in, or inspiration 402, the user 104 of FIG. 1 will experience multiple physiological changes. One change during inspiration 402 will be an increase in heart rate 404 and during expiration there will be a decrease in heart rate 404.

A respiratory-modulated HRV 406 can be detected as a frequency modulation of the multi-wavelength PPG signal and can be derived therefrom. Baroreceptor activities can also modulate the HRV 406; therefore, heart rate variability does not always follow the respiration cycles.

Throughout inspiration 402, a pressure drop 408 can occur with an intrapleural pressure and a right atrial pressure. Next venous return 410 increases leading to an increase in venous blood volume 412.

The increase in venous blood volume 412 can be detected as amplitude fluctuations like respiration-modulated wandering baseline 414. It should be noted that the venous blood volume 412 is also subject to gravity, muscle contraction, and venous compliance; therefore, the wandering baselines 414 are not always in pace with the respiration activities.

After the venous return 410 increases, an end-diastolic volume 416 can increase. Furthermore, a strength of cardiac contraction can increase together with a stroke volume 418.

The increase in the stroke volume 418 results in an arterial blood pulsation 420. The arterial blood pulsation 420 can contain amplitude fluctuations like respiration-modulated pulsation amplitudes 422. It should be noted that the arterial blood pulsation 420 can be also modified by arterial compliance variation; therefore, the respiration modulation in the arterial blood pulsation 420 can be masked.

The heart rate 404, the HRV 406, the venous blood volume 412, the wandering baseline 414, the arterial blood pulsation 420, and the pulsation amplitudes 422 can be understood as the respiratory features 541 of FIG. 5 together with the HRV RR estimate 602 of FIG. 6 and the wandering baseline RR estimate 702 of FIG. 7. Furthermore, the HRV 406, the wandering baseline 414, and the pulsation amplitudes 422 can be respiration modulated.

Referring now to FIG. 5, therein is shown a block diagram of a control flow for the RR prediction system 100. The RR prediction system 100 can initiate the RR prediction by transmitting the multi-wavelength PPG signal into the user 104 of FIG. 1.

While the multi-wavelength PPG signal measurements from optical paths 302, as described with regard to FIG. 3, is contemplated to be the primary embodiment, it is also contemplated that the RR prediction system 100 could have multiple different modes. One contemplated mode is a single wavelength mode, where the RR can be estimated based on the green optical path 306 of FIG. 3 with a heart rate detected simultaneously.

Another contemplated mode is a multi-wavelength mode where the green LED 202 of FIG. 2, the IR LED 206 of FIG. 2, and the red LED 204 of FIG. 2 are enabled for simultaneous RR, heart rate, and oxygen saturation (SpO2) measurement based on green, red and IR PPG signals. Another contemplated mode is a multi-wavelength mode where the IR LED 206 and the red LED 204 are enabled for RR and SpO2 measurement based on red and IR PPG signals.

Continuing with the primary embodiment, the RR prediction system 100 can instruct the multi-wavelength PPG sensor to illuminate one, some, or all of the green LEDs 202, the red LEDs 204, and the IR LEDs 206 and measure the corresponding light intensity in each emitted light wavelength with photodetectors 212, 214, 216 and 218. These multi-wavelength PPG signals 502 can be generated during an emit multi-wavelength PPG signal step 504.

Once the RR prediction system 100 executes the emit multi-wavelength PPG signal step 504, the RR prediction system 100 can execute a collect multi-wavelength PPG signal step 506. During the collect multi-wavelength PPG signal step 506, the RR prediction system 100 can collect the multi-wavelength PPG signals 502 as real-time or buffered hemodynamic signals 508 to an operation window 510 of predefined duration 512 and refresh interval 514.

The multi-wavelength PPG signals 502 can be emitted by the light emitters into the user 104 and are absorbed more or less based on volumetric blood variations within the user 104. The multi-wavelength PPG signals 502 not absorbed within the user 104 can be detected by the photodetectors as the hemodynamic signals 508. The hemodynamic signals 508 are therefore modulated versions of the multi-wavelength PPG signals 502 as well as being a signal modulated by blood volumetric variations.

The operation window 510 can be a sliding window used to collect and analyze hemodynamic signals 508. The operation window 510 can have the duration 512 predefined for 30 seconds and the refresh interval 514 predefined to 10 seconds, for example.

Continuing with this example, the operation window 510 would include 30 seconds of the multi-wavelength PPG signals 502, and every 10 seconds, a new 10 second length of the multi-wavelength PPG signals 502 will be added to one end of the operation window 510 and deleted from the other. The operation windows 510 can be evaluated during a signal quality step 516 with a customized respiratory signal quality indicator 518.

The hemodynamic signals 508 within the operation windows 510 that are not qualified will be rejected and will not be further used. The signal quality step 516 can compute the respiratory signal quality indicator 518 based on multiple signal inputs. One of the multiple signal inputs can be acceleration amplitudes 520 in the X, Y and Z axes.

The acceleration amplitudes 520 can be used to reject the operation window 510, for example if the acceleration amplitudes 520 is above an acceleration threshold 522 at any time during the operation window 510, the operation window 510 can be rejected as motion can degrade the hemodynamic signals 508 detected.

Another one of the multiple signal inputs is a plausible respiration signal 524 extracted from the hemodynamic signals 508 obtained through the IR optical path 310 of FIG. 3. The hemodynamic signals 508 can be filtered to retain a band of 0.15Hz to 0.6Hz, which can be considered the plausible respiration signal 524. A plausible respiration ratio can be calculated as the ratio between the root mean square of the plausible respiration signal 524 and the mean of the unfiltered hemodynamic signals 508 of the IR optical path 310.

The plausible respiration ratio can be used to reject the operation window 510, for example if the plausible respiration ratio is above a plausible respiration threshold, then the operation window 510 will not be rejected. If the plausible respiration ratio is below the plausible respiration threshold, then the operation window 510 containing the hemodynamic signals 508 producing the plausible respiration ratio will be rejected.

Another one of the multiple signal inputs is a plausible cardiac signal 526 extracted from the hemodynamic signals 508 obtained through the green optical path 306. The hemodynamic signals 508 can be filtered to retain a band of 0.6Hz to 5Hz, which can be considered the plausible cardiac signal 526. A plausible cardiac ratio can be calculated as the ratio between the root mean square of the plausible cardiac signal 526 and the mean of unfiltered hemodynamic signals 508 of the green optical path 306.

The plausible cardiac ratio can be used to reject the operation window 510, for example if the plausible cardiac ratio is above a plausible cardiac threshold, then the operation window 510 will not be rejected. If the plausible cardiac ratio is below the plausible cardiac threshold, then the operation window 510 containing the hemodynamic signals 508 producing the plausible cardiac ratio will be rejected.

Other of the multiple signal inputs include green and IR cardiac signal components 528 extracted from the hemodynamic signals 508 obtained through the green optical path 306 and the IR optical path 310. The hemodynamic signals 508 from the green optical path 306 and the IR optical path 310 can each be individually filtered to retain a band of 0.6Hz to 5Hz and can be combined to form a 30 second window within which is computed the Pearson's coefficient.

The Pearson correlation coefficient can be used to reject the operation window 510, for example if the Pearson correlation coefficient is above a Pearson threshold, then the operation window 510 will not be rejected. If the Pearson correlation coefficient is below the Pearson threshold, then the operation window 510 containing the hemodynamic signals 508 producing the plausible cardiac ratio will be rejected.

The plausible respiration signal 524, the plausible cardiac signal 526, and the green and IR cardiac signal components 528 can be calculated in a filter step 530. The plausible respiration signal 524, the plausible cardiac signal 526, and a plausible neurogenic signal can be determined from each wavelength or channel. The channels being different bands of the hemodynamic signals 508 detected from the various optical paths of the multi-wavelength PPG sensor. The RR prediction system 100 can further filter the hemodynamic signals 508, in the filter step 530, to obtain a cardiogenic component 532, a respirogenic component 534, and a neurogenic component 536.

The signal quality step 516 computes the respiratory signal quality indicator 518 which can be understood in reference to a signal threshold 538. The signal threshold 538 can be the plausible respiration threshold, the plausible cardiac threshold, the Pearson threshold, or the acceleration threshold. The respiratory signal quality indicator 518 of the hemodynamic signal 508 can be computed and determined with the acceleration amplitudes 520 from the accelerometer 222, the plausible respiration signal 524, a plausible cardiac signal 526, or a combination thereof by fusing multi-wavelength hemodynamic signals 508.

The fusion can be performed by reconstructing a new signal from principal component analysis (PCA) or independent component analysis on the multi-wavelength hemodynamic signals 508. For example, the PCA can be performed on green, red and IR PPG signals, and the third dimension of PCA could be an orthogonal component to blood pulsations reflecting random tissue motions. This reconstructed signal can facilitate the sensitive detection of motion artifacts and define a respiration signal quality.

If the respiratory signal quality indicator 518 for one of the operation windows 510 falls below the signal threshold 538, then the hemodynamic signals 508 within the operation windows 510 having the low respiratory signal quality indicator 518 will be rejected and will not be further used, including to determine the respiratory features, the signal condition features, the cardiogenic component 532, the respirogenic component 534, or the neurogenic component 536. Because computing the respiratory features, the signal condition features, the cardiogenic component 532, the respirogenic component 534, and the neurogenic component 536 consumes power and computational resources, it provides an improvement to the technology not waste these resources in these computations when the respiratory signal quality indicator 518 for one of the operation windows 510 falls below the signal threshold 538, which limits other prior developments from operating on the wrist or other parts of the body with suboptimal anatomy or vascularity.

The cardiogenic component 532 can be the hemodynamic signals 508 detected from the green optical path 306 and can be filtered to retain signals above 0.65 Hz. The respirogenic component 534 can be the hemodynamic signals 508 detected from the red optical path 308 of FIG. 3 and the IR optical path 310 filtered to retain signals between 0.1 to 0.65 Hz. The neurogenic component 536 can be the hemodynamic signals 508 filtered to keep signals below 0.1 Hz.

Furthermore, the hemodynamic signals 508 from the green optical path 306, the red optical path 308, and the IR optical path 310 to individually compute the cardiogenic component 532, the respirogenic component 534, and the neurogenic component 536 for each color of LED. That is, the hemodynamic signals 508 from the green optical path 306 can be individually filtered to retain signals above 0.65 Hz as a green cardiogenic component, to retain signals between 0.1 to 0.65 Hz as a green respirogenic component, and to retain signals below 0.1 Hz as a green neurogenic component.

The hemodynamic signals 508 from the red optical path 308 can be individually filtered to retain signals above 0.65 Hz as a red cardiogenic component, to retain signals between 0.1 to 0.65 Hz as a red respirogenic component, and to retain signals below 0.1 Hz as a red neurogenic component. The hemodynamic signals 508 from the IR optical path 310 can be individually filtered to retain signals above 0.65 Hz as an IR cardiogenic component, to retain signals between 0.1 to 0.65 Hz as an IR respirogenic component, and to retain signals below 0.1 Hz as an IR neurogenic component.

While the cardiogenic component 532, the respirogenic component 534, and the neurogenic component 536 are described as being filtered from different frequency bands, channels, or wavelengths of light, it is contemplated that the cardiogenic component 532, the respirogenic component 534, and the neurogenic component 536 can be determined from each wavelength channel or for each different frequency band of the hemodynamic signals 508. After determining the cardiogenic component 532, the respirogenic component 534, and the neurogenic component 536 in the filter step 530, the RR prediction system 100 can execute a feature extraction step 540. During the feature extraction step 540, the RR prediction system 100 can determine respiratory features 541 including the HRV 406, the wandering baseline 414, the HRV RR estimate 602 of FIG. 6, and the wandering baseline RR estimate 702 of FIG. 7.

The HRV 406 can be determined from the green optical path 306 component of the hemodynamic signals 508. The HRV 406 can represent respiratory sinus arrhythmia, which is the HRV 406 of the user 104 synchronized with the respiration of the user 104.

The wandering baseline 414 can be determined from the IR optical path 310 and the red optical path 308 components of the hemodynamic signals 508. The wandering baseline 414 can represent the impact of lung pumping activities on blood volume of the user 104.

The HRV 406 and the wandering baseline 414 can be further processed in a peak and cycle detection step 542, which is further described in FIGs. 6 and 7 below. The HRV RR estimate 602 and the wandering baseline RR estimate 702 can be calculated in a RR estimation step 544.

It has been discovered that discrepancies between the HRV RR estimate 602 and the wandering baseline RR estimate 702 can be calculated during RR estimation step 544. The discrepancies can be differences between the HRV RR estimate 602 and the wandering baseline RR estimate 702 generated by the differing physical properties of the green optical path 306 and the IR optical path 310, for example, and gives rise to an uncertainty of which estimate is closest to the actual RR.

During the feature extraction step 540, the RR prediction system 100 can also calculate signal condition features 545 that reflect the balance between the cardiovascular and respiratory modulations in the body of the user 104. More particularly, the signal condition features 545 can reflect confounding factors modulating the PPG waveform and respiration activities, such as the local perfusion, neural-modulated vasomotion, and muscle contractions. These signal condition features 545 can include a cardio-respiratory balance 546, a neuro-respiratory balance 548, a green cardio-respiratory coupling 550, an IR cardio-respiratory coupling 552, or a combination thereof by fusing the morphological and statistical features of multi-wavelength hemodynamic signals 508.

Morphological and statistical features can be the statistical metrics to reflect the pulsatility of the signal, such as the variance, root mean square, and peak-to-average ratio of the signal segment. Fusing methods can be defining an indicator as the ratios or a weighted summation of those aforementioned features.

Furthermore, the RR prediction system 100 can implement a feature selection step 554 to select which of the signal condition features 545 should be selected for calculation and which should not be. The selection of the signal condition features 545 can be changed during the feature extraction step 540.

The feature selection step 554 can select one or multiple of the signal condition features 545 for calculation. The feature selection step 554 can consider the power consumption of calculating the signal condition feature 545, the computation burden of calculating the signal condition feature 545, and the model complexity of calculating the signal condition feature 545 when determining whether or not to calculate the signal condition feature 545. The power consumption, the computation burden, and the model complexity for each signal condition feature 545 can be evaluated against a threshold for that parameter when deciding whether or not to compute the signal condition feature 545. Therefore, the feature selection step 554 provides a real-time physiological-informed feature selection based on the hemodynamic signals 508 which simultaneously mitigates uncertainty in RR predictions, reduces power consumption, and reduces computational recourses needed.

The cardio-respiratory balance 546 can be determined by filtering the portion of the hemodynamic signals 508 obtained through the green optical path 306 with a cardiac passband filter 556 and retaining green cardiac signal components over 0.65 Hz. Furthermore, the hemodynamic signals 508 obtained through the IR optical path 310 are filtered with the cardiac passband filter 556 and retain IR cardiac signal components over 0.65 Hz.

The cardio-respiratory balance 546 can be calculated as the correlation coefficient between the green cardiac signal components and the IR cardiac signal components. It has been discovered that the pulsation amplitude of IR cardiac signal component is more related to respiration-induced changes on arterial blood volume than the Green cardiac signal component.

The neuro-respiratory balance 548 can be determined by filtering the portion of the hemodynamic signals 508 obtained through the green optical path 306 with a respiratory passband filter 558 and retaining green respiratory signal components between 0.1 Hz and 0.65 Hz. Furthermore, the hemodynamic signals 508 obtained through the IR optical path 310 are filtered with the respiratory passband filter 558 and retain IR respiratory signal components between 0.1 Hz and 0.65 Hz. The neuro-respiratory balance 548 can be calculated as the correlation coefficient between the green respiratory signal components and the IR respiratory signal components.

The green cardio-respiratory coupling 550 can be determined by filtering the portion of the hemodynamic signals 508 obtained through the green optical path 306 with the cardiac passband filter 556 and retaining green cardiac signal components over 0.65 Hz. The green cardiac signal components can be analyzed to detect peaks and calculate inter-heartbeat intervals within the operation window 510.

It is contemplated that the operation window 510 can be preferably longer than 30 seconds for calculating the green cardio-respiratory coupling 550. Once the inter-heartbeat intervals are calculated, the RR prediction system 100 can calculate the standard deviation of the inter-heartbeat intervals as the green cardio-respiratory coupling 550.

The IR cardio-respiratory coupling 552 can be determined by filtering the portion of the hemodynamic signals 508 obtained through the IR optical path 310 with the respiratory passband filter 558 and retaining IR respiratory signal components between 0.1 Hz and 0.65 Hz. Furthermore, the hemodynamic signals 508 obtained through the IR optical path 310 are filtered with the cardiac passband filter 556 and retain IR cardiac signal components over 0.65 Hz. The IR cardio-respiratory coupling 552 can be calculated as the ratio of the root mean square of the IR respiratory signal component to the root mean square of IR cardiac signal component.

The respiratory features 541 can include: the HRV RR estimate 602 and the wandering baseline RR estimate 702 of FIG. 7 from multi-wavelength hemodynamic signals 508. The signal condition features 545 can include: the cardio-respiratory balance 546, the neuro-respiratory balance 548, the green cardio-respiratory coupling 550, and the IR cardio-respiratory coupling 552 can be provided as inputs to a machine learning algorithm 560. Biometric data 562 including age, weight, sex, height, and others can also be provided as inputs to the machine learning algorithm 560.

The signal condition features 545, as input features to the machine learning algorithm 560, can provide supplementary information for the machine learning algorithm 560. The machine learning algorithm 560 can utilize the signal condition features 545 to adjust the weights of RR estimates, or to serve as decision nodes for choosing one or a subset average for the RR estimates, thus mitigating the uncertainty in final RR prediction 564.

The machine learning algorithm 560 can be a linear regression or a Gaussian process regression or neural network model. In one contemplated embodiment, the k-fold cross-validation can be used be for model performance evaluation. It is contemplated that other forms of training could be implemented such as hyper-parameter tuning.

Consistent cross-validation results have been observed from two studies having separate datasets with different age distributions and intervention maneuvers. When the signal condition features 545 were included as inputs to the machine learning algorithm 560 together with the respiratory features 541 such as HRV RR estimates 602 and wandering baseline RR estimate 702, the correlation between the final RR prediction 564 and reference RR improves and the root mean squared error (RMSE) reduces.

Testing both the Gaussian process regression model against the linear regression model resulted in the Gaussian process regression outperforming the linear regression model and reaching the target accuracy of RMSE < 3 respirations per minute from a wrist worn optical sensing device. These results demonstrated the technical improvement over prior developments in the ability to accurately predict RR on areas of the body with suboptimal vasculature.

Furthermore, the added value of including the signal condition features 545 together with the HRV RR estimate 602 and the wandering baseline RR estimate 702 was shown by improvement in the RMSE and the correlation between the final RR prediction 564 and the reference RR. Thus, it has been discovered that utilizing the signal condition features 545 together with the Gaussian process regression achieves a clinical-grade accuracy in RR monitoring from areas of the body with suboptimal vasculature.

The output of the machine learning algorithm 560 can be the RR prediction 564 and a confidence score 566. The RR prediction 564 can be the result of the Gaussian process regression on the inputs to the machine learning algorithm 560. The confidence score 566 can be based on the HRV RR estimate 602, the wandering baseline RR estimate 702, the signal condition features 545, or a combination thereof being inputs into the machine learning algorithm 560.

The Gaussian process regression is a non-parametric Bayesian approach that provides uncertainty estimates for its predictions. The Gaussian process regression model is suitable to handle the multi-wavelength PPG signal based RR predictions 564, considering the uncertainty in divergent RR predictions 564 from different optical paths 302 and the covariances with the physiological coupling.

The RR prediction 564 can be directly provided by the HRV RR estimate 602 and the wandering baseline RR estimate 702 depending on the operation modes of the RR prediction system 100 considering the computation and power consumption. For example, the RR prediction 564 could be augmented by being averaged with the HRV RR estimate 602 and the wandering baseline RR estimate 702 from one or multiple wavelength PPG signals.

As will be appreciated, the Gaussian process regression not only provides the RR prediction 564 but also provides the confidence score 566 as a quantification of uncertainty for the prediction. The RR prediction system 100 can operate to output, or not, the prediction to user based on the confidence score 566 as a tuning between measurement coverage and accuracy; which improves the technology by overcoming the occasionally inaccurate data from the wrist or other parts of the body with suboptimal anatomy or vascularity and which prevent other, prior developments, from obtaining accurate readings.

The confidence score 566 can be understood in relation to a confidence threshold 568. The confidence threshold 568 can be configured to determine whether to output the RR prediction 564 from the machine learning algorithm 560 or not. Illustratively, if the confidence score 566 falls below the confidence threshold 568, the RR prediction 564 will not be output from the machine learning algorithm 560 and the RR prediction 564 corresponding to the low confidence score 566 will be deleted. However, if the confidence score 566 exceeds the confidence threshold 568, the RR prediction 564 will be output from the machine learning algorithm 560.

Referring now to FIG. 6, therein is shown a block diagram of a control flow for determining an HRV RR estimate 602. The HRV RR estimate 602 can be a respiration-modulated HRV RR estimate. The RR prediction system 100 can begin by initiating the collect multi-wavelength PPG signal step 506.

All of the multi-wavelength PPG signals could be collected in the collect multi-wavelength PPG signal step 506; however, it is contemplated that the hemodynamic signals 508 of FIG. 5 from the green optical path 306 of FIG. 3 are collected for determining the HRV RR estimate 602. The hemodynamic signals 508 from the green optical path 306 can be filtered in a band pass filter step 604. During the band pass filter step 604, the hemodynamic signals 508 from the green optical path 306 can be filtered to retain signals between 0.5 Hz and 10 Hz.

The filtered signal can then be input into a heartbeat peak detection step 606, during which the peaks or other fiducial points of the filtered signal can be detected to mark a cardiac cycle. The horizontal space representing time and a time interval between heartbeat peaks 608 can be computed as the heart rate 404 of FIG. 4.

Once the heartbeat peak detection step 606 has completed and any heartbeat peaks 608 have been identified, the RR prediction system 100 can execute a heartbeat detection decision step 610. During the heartbeat detection decision step 610, the RR prediction system 100 can determine whether any of the heartbeat peaks 608 have been detected, if so the heartbeat detection decision step 610 can return a positive result and the RR prediction system 100 can execute a beat-by-beat respiration peak detection step 612.

If the heartbeat detection decision step 610 does not detect the heartbeat peaks 608, then a negative result is returned and the collect multi-wavelength PPG signal step 506 can be re-executed. During the beat-by-beat respiration peak detection step 612, the heart rate at each beat of the heart can be computed.

That is, a beat-by-beat heart rate 614 can first be computed. The beat-by-beat heart rate 614 is depicted with time along the horizontal axis and heart rate along the vertical axis, i.e., beat to beat heart rate variation as a function of time. A faster heartbeat will plot at a higher vertical position while a lower heartbeat will plot vertically lower.

The peaks, or local maximum of the beat-by-beat heart rate 614 can be determined as respiration peaks 616 using a time-domain approach to detect the respiration peaks 616. The respiration peaks 616 simultaneously correspond both to a higher HR and a breath in and the number of the respiration peaks 616 over time can be transformed to the HRV RR estimate 602 in the unit of respirations per minute. Once any respiration peaks 616 are determined during the beat-by-beat respiration peak detection step 612, the RR prediction system 100 can execute a respiration detection decision step 618.

During the respiration detection decision step 618 the RR prediction system 100 can determine whether any of the respiration peaks 616 have been detected. If so, the respiration detection decision step 618 can return a positive result and the RR prediction system 100 can execute an update HRV RR estimate step 620.

If the respiration detection decision step 618 does not detect the respiration peaks 616, then a negative result is returned and the collect multi-wavelength PPG signal step 506 can be re-executed. The beat-by-beat respiration peak detection step 612 can implement a time-domain approach to detect the respiration peaks 616.

Once the respiration peaks 616 are detected, the HRV RR estimate 602 is computed as the number of the respiration peaks 616 over time. The HRV RR estimate 602 is updated during the update HRV RR estimate step 620.

Alternatively, the HRV RR estimate 602 can be determined by computing a frequency domain spectrum of the HRV 406 of FIG. 4. The dominant frequency peak in the frequency domain spectrum can be recognized and updated as the HRV RR estimate 602.

Referring now to FIG. 7, therein is shown a block diagram of a control flow for determining a wandering baseline RR estimate 702. The wandering baseline RR estimate 702 can be a respiration-modulated wandering baseline RR estimate 702. The RR prediction system 100 can begin by initiating the collect multi-wavelength PPG signal step 506.

All of the multi-wavelength PPG signals could be collected in the collect multi-wavelength PPG signal step 506; however, it is contemplated that the hemodynamic signals 508 of FIG. 5 from the red optical path 308 of FIG. 3 and the IR optical path 310 of FIG. 3 are be collected for determining the wandering baseline RR estimate 702.

The hemodynamic signals 508 from the red optical path 308 and IR optical path 310 can be filtered in a band pass filter step 704. During the band pass filter step 704, the hemodynamic signals 508 from the red optical path 308 and the IR optical path 310 can be filtered to retain signals below 0.5 Hz.

Baseline wandering can be isolated from the filtered signal as a DC or slowly varying component of the filtered signal. The DC component of the filtered signal can then be input into a respiration peak detection step 712. During the respiration peak detection step 712, respiration inducing larger blood volumes within the vasculature corresponding to a breath in can be detected.

That is, respiration peaks 714 within the DC component of the filtered signal can be detected using a time-domain approach to detect the respiration peaks 714. The number of the respiration peaks 714 over time can be the respiration rate 716 in the unit of respirations per minute. Once any respiration peaks 714 are determined during the respiration peak detection step 712, the RR prediction system 100 can execute a respiration detection decision step 718.

During the respiration detection decision step 718 the RR prediction system 100 can determine whether any of the respiration peaks 714 have been detected. If so, the respiration detection decision step 718 can return a positive result and the RR prediction system 100 can execute an update wandering baseline RR estimate step 720.

If the respiration detection decision step 718 does not detect the respiration peaks 714, then a negative result is returned and the collect multi-wavelength PPG signal step 506 can be re-executed. The respiration peak detection step 712 can implement a time-domain approach to detect the respiration peaks 714.

Once the respiration peaks 714 are detected the wandering baseline RR estimate 702 is measured as the number of the respiration peaks 714 over time. The wandering baseline RR estimate 702 is updated during the update wandering baseline RR estimate step 720.

Thus, it has been discovered that the RR prediction system 100 can meet industry needs of providing RR monitoring in a watch and achieve accuracy in RMSE below 3 respirations per minute. That is, the collect multi-wavelength PPG signal step 506, the signal quality step 516, the filter step 530, and the feature extraction step 540, all of FIG. 5, which determine, filter, calculate, and analyze the respiratory features 541 and the signal condition features 545, which can be used as inputs to the machine learning algorithm 560 implemented as a Gaussian process regression model, reaches the target accuracy of RMSE < 3 respirations per minute from a wrist worn optical sensing device.

The RR prediction system therefore furnishes important and heretofore unknown and unavailable solutions, capabilities, and functional aspects over prior developments in the RR detection technologies. The resulting configurations are straightforward, cost-effective, uncomplicated, highly versatile, accurate, sensitive, and effective, and can be implemented by adapting known components for ready, efficient, and economical manufacturing, application, and utilization.

While the RR prediction system has been described in conjunction with a specific best mode, it is to be understood that many alternatives, modifications, and variations will be apparent to those skilled in the art in light of the preceding description. Accordingly, it is intended to embrace all such alternatives, modifications, and variations, which fall within the scope of the included claims. All matters set forth herein or shown in the accompanying drawings are to be interpreted in an illustrative and non-limiting sense.

There follows a list of numbered features defining particular embodiments of the present disclosure. Where a numbered feature refers to one or more earlier numbered features then those features should be considered in combination.
1. A respiration rate prediction method comprising:
   emitting light into tissue with a light emitter;
   detecting the light from the tissue with a photodetector as a hemodynamic signal;
   determining a respiration rate estimate and a signal condition feature from the hemodynamic signal;
   determining a signal quality indicator from the signal condition feature;
   deciding whether to determine a respiration rate prediction with a machine learning algorithm based on the signal quality indicator;
   determining the respiration rate prediction and a confidence score for the respiration rate prediction, the confidence score based on the respiration rate estimate and the signal condition feature being inputs into the machine learning algorithm; and
   outputting the respiration rate prediction from the machine learning algorithm based on the confidence score.
2. The method of feature 1 wherein emitting the light includes emitting green light, red light, infrared light, or a combination thereof.
3. The method of feature 1 or 2, wherein determining the respiration rate estimate includes determining the respiration rate estimate based on a respiration-modulated heart rate variability respiration rate estimate, a respiration-modulated wandering baseline respiration rate estimate, a respiration-modulated pulsation amplitude, or a combination thereof.
4. The method of any of the preceding features, wherein determining the respiration rate estimate and the signal condition feature from the hemodynamic signal includes determining a neurogenic component, a respirogenic component, and a cardiogenic component, and wherein the neurogenic component, the respirogenic component, and the cardiogenic component are each determined from different wavelength channels of the hemodynamic signal.
5. The method of any of the preceding features, wherein determining the signal condition feature includes determining a cardio-respiratory balance, a neuro-respiratory balance, or a combination thereof by fusing morphological or statistical features of the hemodynamic signal.
6. The method of any of the preceding features, wherein determining the signal quality indicator includes determining the signal quality indicator with an acceleration amplitude, a plausible respiration signal, a plausible cardiac signal, or a combination thereof by fusing multi-wavelength signals within the hemodynamic signal.
7. The method of any of the preceding features, wherein determining the respiration rate prediction includes determining the respiration rate prediction based on biometric data being input into the machine learning algorithm.
8. The method of any of the preceding features, wherein determining the respiration rate prediction includes determining the respiration rate prediction with a Gaussian process regression algorithm.
9. The method of any of the preceding features, wherein outputting the respiration rate prediction includes outputting the respiration rate prediction based on the confidence score exceeding a confidence threshold.
10. A non-transitory computer readable medium in useful association with a processor having instructions configured to:
   emit light into tissue with a light emitter;
   detect the light from the tissue with a photodetector as a hemodynamic signal;
   determine a respiration rate estimate and a signal condition feature from the hemodynamic signal;
   determine a signal quality indicator from the signal condition feature;
   decide whether to determine a respiration rate prediction with a machine learning algorithm based on the signal quality indicator;
   determine the respiration rate prediction and a confidence score for the respiration rate prediction, the confidence score based on the respiration rate estimate and the signal condition feature being inputs into the machine learning algorithm; and
   output the respiration rate prediction from the machine learning algorithm based on the confidence score.
11. The computer readable medium of feature 10 wherein the instructions configured to emit the light includes instructions configured to emit green light, red light, infrared light, or a combination thereof.
12. The computer readable medium of feature 10 or 11 wherein the instructions configured to determine the respiration rate estimate include instructions configured to determine the respiration rate estimate based on a respiration-modulated heart rate variability respiration rate estimate, a respiration-modulated wandering baseline respiration rate estimate, a respiration-modulated pulsation amplitude, or a combination thereof.
13. The computer readable medium of any of features 10 to 12 wherein the instructions configured to determine the respiration rate estimate and the signal condition feature from the hemodynamic signal includes instructions configured to determine a neurogenic component, a respirogenic component, and a cardiogenic component, and wherein the neurogenic component, the respirogenic component, and the cardiogenic component are each determined from different wavelength channels of the hemodynamic signal.
14. The computer readable medium of any of features 10 to 13, wherein the instructions configured to determine the signal condition feature include instructions configured to determine a cardio-respiratory balance, a neuro-respiratory balance, or a combination thereof by fusing morphological or statistical features of the hemodynamic signal.
15. The computer readable medium of any of features 10 to 14, wherein the instructions configured to determine the signal quality indicator of the hemodynamic signal include instructions configured to determine the signal quality indicator with an acceleration amplitude, a plausible respiration signal, a plausible cardiac signal, or a combination thereof by fusing multi-wavelength signals within the hemodynamic signal.
16. The computer readable medium of any of features 10 to 15, wherein the instructions configured to determine the respiration rate prediction include instructions configured to determine the respiration rate prediction based on biometric data being input into the machine learning algorithm.
17. The computer readable medium of any of features 10 to 16, wherein the instructions configured to determine the respiration rate prediction include instructions configured to determine the respiration rate prediction with a Gaussian process regression algorithm.
18. The computer readable medium of any of features 10 to 17, wherein the instructions configured to output the respiration rate prediction includes instructions configured to output the respiration rate prediction based on the confidence score exceeding a confidence threshold.
19. A respiration rate prediction system comprising:
   a light emitter configured to emit light into tissue;
   a photodetector configured to detect the light as a hemodynamic signal; and
   a processor configured to:
      determine a respiration rate estimate and a signal condition feature from the hemodynamic signal,
      determine a signal quality indicator from the signal condition feature,
      decide whether to determine a respiration rate prediction with a machine learning algorithm based on the signal quality indicator,
      determine the respiration rate prediction and a confidence score for the respiration rate prediction, the confidence score based on the respiration rate estimate and the signal condition feature being inputs into the machine learning algorithm, and
      output the respiration rate prediction from the machine learning algorithm based on the confidence score.
20. The system of feature 19 wherein the light emitter is configured to emit green light, red light, infrared light, or a combination thereof.
21. The system of feature 19 or 20, wherein the processor is configured to determine the respiration rate estimate is configured to determine the respiration rate estimate based on a respiration-modulated heart rate variability respiration rate estimate, a respiration-modulated wandering baseline respiration rate estimate, a respiration-modulated pulsation amplitude, or a combination thereof.
22. The system of any of features 19 to 21, wherein the processor is configured to determine the respiration rate estimate and the signal condition feature from the hemodynamic signal is configured to determine a neurogenic component, a respirogenic component, and a cardiogenic component, and wherein the neurogenic component, the respirogenic component, and the cardiogenic component are each determined from different wavelength channels of the hemodynamic signal.
23. The system of any of features 19 to 22, wherein the processor is configured to determine the signal condition feature is configured to determine a cardio-respiratory balance, a neuro-respiratory balance, or a combination thereof by fusing morphological or statistical features of the hemodynamic signal.
24. The system of any of features 19 to 23, wherein the processor is configured to determine the signal quality indicator of the hemodynamic signal is configured to determine the signal quality indicator with an acceleration amplitude, a plausible respiration signal, a plausible cardiac signal, or a combination thereof by fusing multi-wavelength signals within the hemodynamic signal.
25. The system of any of features 19 to 24, wherein the processor is configured to determine the respiration rate prediction is configured to determine the respiration rate prediction based on biometric data being input into the machine learning algorithm.
26. The system of any of features 19 to 25, wherein the processor is configured to determine the respiration rate prediction is configured to determine the respiration rate prediction with a Gaussian process regression algorithm.
27. The system of any of features 19 to 26, wherein the processor is configured to output the respiration rate prediction is configured to output the respiration rate prediction based on the confidence score exceeding a confidence threshold.

## Claims

1. A respiration rate prediction method comprising:
emitting light into tissue with a light emitter;
detecting the light from the tissue with a photodetector as a hemodynamic signal;
determining a respiration rate estimate and a signal condition feature from the hemodynamic signal;
determining a signal quality indicator from the signal condition feature;
deciding whether to determine a respiration rate prediction with a machine learning algorithm based on the signal quality indicator;
determining the respiration rate prediction and a confidence score for the respiration rate prediction, the confidence score based on the respiration rate estimate and the signal condition feature being inputs into the machine learning algorithm; and
outputting the respiration rate prediction from the machine learning algorithm based on the confidence score.

2. The method of claim 1 wherein emitting the light includes emitting green light, red light, infrared light, or a combination thereof.

3. The method of claim 1 or 2, wherein determining the respiration rate estimate includes determining the respiration rate estimate based on a respiration-modulated heart rate variability respiration rate estimate, a respiration-modulated wandering baseline respiration rate estimate, a respiration-modulated pulsation amplitude, or a combination thereof.

4. The method of any of the preceding claims, wherein determining the respiration rate estimate and the signal condition feature from the hemodynamic signal includes determining a neurogenic component, a respirogenic component, and a cardiogenic component, and wherein the neurogenic component, the respirogenic component, and the cardiogenic component are each determined from different wavelength channels of the hemodynamic signal.

5. The method of any of the preceding claims, wherein determining the signal condition feature includes determining a cardio-respiratory balance, a neuro-respiratory balance, or a combination thereof by fusing morphological or statistical features of the hemodynamic signal.

6. The method of any of the preceding claims, wherein determining the signal quality indicator includes determining the signal quality indicator with an acceleration amplitude, a plausible respiration signal, a plausible cardiac signal, or a combination thereof by fusing multi-wavelength signals within the hemodynamic signal.

7. The method of any of the preceding claims, wherein determining the respiration rate prediction includes determining the respiration rate prediction based on biometric data being input into the machine learning algorithm.

8. The method of any of the preceding claims, wherein determining the respiration rate prediction includes determining the respiration rate prediction with a Gaussian process regression algorithm.

9. The method of any of the preceding claims, wherein outputting the respiration rate prediction includes outputting the respiration rate prediction based on the confidence score exceeding a confidence threshold.

10. A non-transitory computer readable medium storing computer-readable instructions that when executed by a processor cause the processor to perform the method of any of the preceding claims.

11. A respiration rate prediction system comprising:
a light emitter configured to emit light into tissue;
a photodetector configured to detect the light as a hemodynamic signal; and
a processor;
wherein the system is further configured such that in use it performs the method of any of claim 1 to 9.
29.
